# EUROPEAN PATENT SPECIFICATION

(11) **EP 4 536 156 B1**
(45) Date of publication and mention of the grant of the patent: **22.07.2026**
(21) Application number: 23732175.7
(22) Date of filing: 06.06.2023
(51) Int. Cl.: A61F 5/37, A61F 5/03

(54) **POST-SURGICAL THORACIC SUPPORT VEST**
WESTE ZUR POSTCHIRURGISCHEN THORAXUNTERSTÜTZUNG
GILET DE SOUTIEN THORACIQUE POST-CHIRURGICAL

(30) Priority: 07.06.2022 GB 202208319
(43) Date of publication of application: 16.04.2025
(73) Proprietor: Guy's and St Thomas' NHS Foundation Trust, London SE1 9RT (GB)
(72) Inventor: ROCHON, Melissa, Harefield Middlesex UB9 6JH (GB); MORAIS, Carlos, Westminster Bridge Rd London SE1 7EH (GB); HUTTON, Sandra, Oxford Oxfordshire OX3 9DU (GB); ROSALIE, Magboo, London EC1A 7BE (GB)
(74) Representative: McDougall, James
(86) International application number: PCT/GB2023/051476
(87) International publication number: WO 2023/237872

(56) References cited:
- US-A1- 2016 113 808
- US-B1- 6 830 050

## Description

### Field of the Invention

The present invention relates to a medical device which is a post-surgical thoracic support vest comprising a resilient body and patient operable fastening arrangement.

### Background

In the field of post-operative recovery, there is a need to support the chest cavity (thorax) of patients after chest surgery. A literature review by Vos et al. (2018)¹ included two studies and reported post-operative chest support using a vest or corset may be effective in reducing the risk of deep sternal wound infection. Numerous post-operative chest support systems exist, including devices made by Posthorax, Sternasafe and Qualibreath. However, all the existing systems suffer from certain disadvantages which have yet to be overcome.

In particular, the prior art devices suffer from poor comfort for some patients, leading to a significant proportion of patients refusing to wear the support system. Trials involving the use of vests report relatively high rates of participant's non-compliance (approximately 15%), often due to discomfort or the device slipping, particularly in female patients (Gorlitzer et al, (2013)², Celik et al., (2011)³). Women in particular find the existing systems uncomfortable to wear due to being inflexible and not designed for a female body shape.

The prior art devices also suffer from a lack of flexibility, and risk of infection of the wound area. For example, the chest support system made by Posthorax resembles a lifejacket and is secured at the front with a clip. This device and others like it have very limited flexibility, due to their design and the materials from which they are made. This can lead to them feeling tight across the chest and being uncomfortable to wear, particularly for long periods. They are also difficult and time consuming to adjust and in general the patient cannot adjust the device themselves. In general, once such a device has been set by adjusting they exhibit no stretch and recovery. The ability to adjust can be particularly important post chest surgery, where the patient may initially retain a large amount of fluid, which they gradually lose.

Having the clips or other fastening features at the front of the device also leads to an increased risk of infection of the wound site, since this is typically over the sternum. Fastening and un-fastening the device brings the patient's or healthcare professional's hands very close to the wound site, increasing the risk of contact and infection. Due to the materials they are made of, existing systems can typically only be washed at low temperatures, which presents a further hygiene risk.

Elastic or long stretch bandages can sustain applied (high) pressure for extended time periods, but may cause unpleasant feelings in the resting, sitting or lying positions. There is also a risk that the bandage may be applied too tightly and cause damage. Adjustment of the bandage is also time consuming, increases the risk of infection each time it is done and cannot be done by the patient. Document US 2016/113808 describes a post-surgical thoracic support vest according to the preamble of claim 1.

### Summary of the Invention

A first aspect of the invention provides a post-surgical thoracic support vest according to claim 1.

Other embodiments are covered by claims 2-11.

### Brief Description of the Figures

So that the general concepts set out in the foregoing sections can be more fully understood, embodiments thereof will be described with reference to the accompanying drawings, in which:
Figure 1a shows a CAD drawing of a support vest according to an embodiment;
Figure 1b shows the same support vest as in Figure 1a with dimension arrows and references as listed in table 1; and
Figure 2 shows an image of the support vest of Figure 1a being demonstrated.

### Detailed description

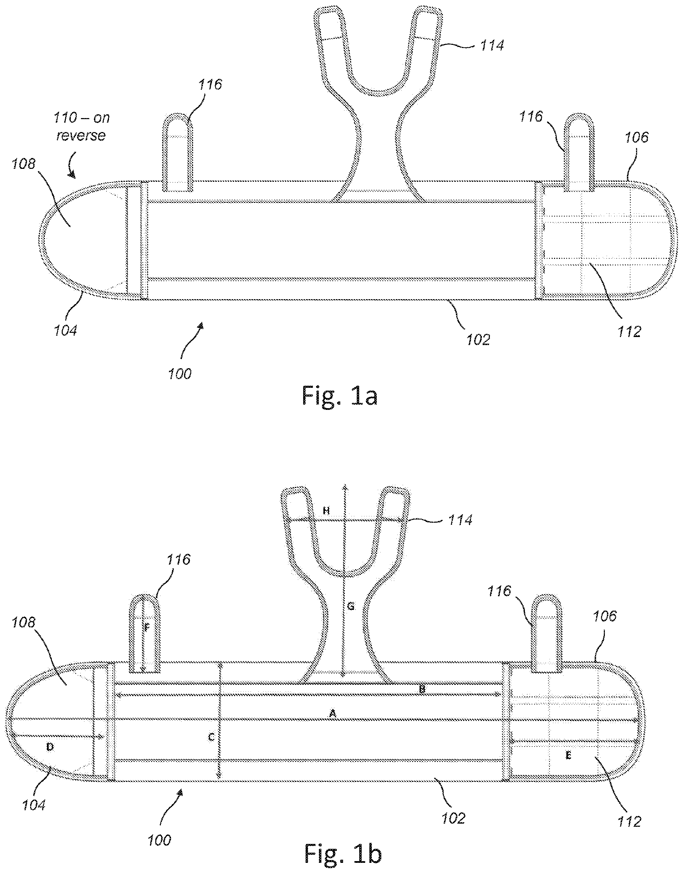

Referring to Figure 1a, a support vest 100 is shown. The support vest 100 is a post-surgical or post-operative thoracic support vest 100 designed to provide pressure to support the thorax of a patient (for example to keep the sternum closed) while they are recovering from chest surgery. The support vest 100 is suitable for use after surgery where a central chest incision has been made and in other therapeutic situation where the chest may need support, for example after rib fracture.

The support vest 100 comprises a resilient central elongate portion 102, a first peripheral (or side) portion 104 and a second peripheral (or side) portion 106. The central elongate portion 102 may be generally rectangular in shape. The longer dimension of the central elongate portion 102 defines the elongate direction of the central elongate portion 102 and of the support vest 100 in general. When stretched beyond its resting dimensions in the elongate direction, the central elongate portion 102 is placed under tensions and exerts a return force. The central elongate portion 102 is configured to be wrapped around the thorax of a patient. When wrapped around the thorax of a patient this force is felt as a "hug" or constricting force directed inwards. This force is produced over a wide range of dimensions whenever the central elongate portion 102 is stretched beyond its resting dimensions in the elongate direction, meaning that the support vest 100 can is effective for patients of different chest sizes with no set-up required. The central elongate portion 102 has an inner surface which contacts the patient when the patient wears the support vest 100 and an outer surface, which faces away from the patient.

The first peripheral portion 104 and the second peripheral portion 106 are disposed at first and second ends of the central elongate portion 102 respectively and may be substantially square or rectangular with a rounded or semi-circular end. Alternatively, the first peripheral portion 104 and second peripheral portion 106 may be semi-circular, U-shaped or parabola shaped.

In the embodiment shown in Figure 1a, the first peripheral portion 104 is U-shaped and has a pocket 108. The pocket 108 is formed from an additional piece of material sown onto the outer surface of the first peripheral portion 104, such that the first peripheral portion 104 resembles a glove or mitt when viewed from the outer surface. The pocket 108 is arranged so as to allow the patient to insert at least a part of a hand into the pocket. The opening of the pocket 108 is arranged perpendicular to the elongate direction of the central elongate portion 102. This arrangement means that the opening of the pocket is located so as to allow the patient to comfortably insert their hand into the pocket 108 while wearing the support vest 100, meaning the patient can use and adjust the support vest with no or minimal assistance.

The inner surface (not visible) of the first peripheral portion 104 supports an attachment surface 110. The attachment surface 110 comprises one half of a contact or touch based fastening arrangement. The other half of the contact or touch based fastening arrangement is formed by a co-operating attachment surface 112 provided on the outer surface of the second peripheral portion 106. The two halves of the contact or touch based fastening arrangement are designed to releasably secure the first peripheral portion 104 and second peripheral portion 106 together when touched together, so as to secure the support vest 100 to the patient. Minimal downward force is required to effect the fastening of the two attachment surfaces. The positioning of the pocket 108 also allows the patient to attach and release the attachment surfaces using a single hand while wearing the vest.

The contact or touch based fastening arrangement may be a hook and loop fastener, such as those made by Velcro^{®}. Alternatively, a different type of fastening arrangement may be used which fastens on contact between the two attachment surfaces, but which can be released by pulling upwards on the first peripheral portion 104 using the pocket 108. For example the Dual Lock^{™} system made by 3M. Using a fastening arrangement which fastens easily on contact between the two halves is advantageous for several reasons:
1. It is easy for the patient to fasten and un-fasten the touch based fastening arrangement, even if they have poor dexterity. The patient does not therefore need to employ any fine motor skills to fasten and unfasten the support vest 100, and can do so without assistance.
2. The patient or healthcare professional may also set the position of the first peripheral portion 104 on top of the second peripheral portion 106, and therefore the amount of support provided by the support vest 100, with a high degree of control and without the need to adjust any straps or buckles. This ensures that the support vest 100 is comfortable for the patient to wear for long periods and provides a correct and precise level of hold force.
3. The process of adjusting the position of the fastening arrangement is also very quick and intuitive for the patient, which not only ensure that the hold force provide by the support vest 100 is appropriate, but encourages the patient to adjust the support vest 100 to improve comfort or to increase/decrease the hold force as necessary, for example as the patient loses or gains fluid post-surgery.

In some embodiments, the first peripheral portion 104 and second peripheral portion 106 are constructed from separate pieces of material and are permanently fixed to the ends of the central elongate portion 102 during manufacture. Where the central elongate portion 102 wraps completely, or almost completely around the thorax of the patient, it is not necessary for the first peripheral portion 104 and the second peripheral portion 106 to be made of a resilient material. However, in some other embodiments, the first peripheral portion 104 and second peripheral portion 106 are integral with the central elongate portion 102, i.e. constructed from the same piece of material, and only the addition of the pocket 108, attachment surface 110 and co-operating attachment surface 112 distinguish the peripheral portions from the central portion.

Constructing the main part of the support vest 100 from a resilient material is advantageous since the vest then provides a homogenous support force over the whole area covered, leading to greatly improved comfort compared to an inflexible supporting structure. The force is distributed evenly across the support vest 100 and around the patient's thorax, in contrast to some existing support garments, in which the action is focussed at the front of the garment. For example, if the patient changes position from sitting to lying, the support force provided at the front of the vest does not substantially change, since it is provided by the resilience of the material at that location. This is not necessarily the case for a more rigid support system, where lying down may compress the patient's rib cage and/or back tissue causing the support force provided by the more rigid support system at the front of the patient to change, e.g. reduce. This is undesirable, particularly since the support vest is designed to be used after chest surgery where an incision has been made in the sternum, meaning that this area in particular requires consistent support. Prior art support systems are also known to be poorly designed and uncomfortable for females. The resilient nature of the support vest 100 alleviates this issue by providing a vest which is comfortable to wear and which provides a consistent support force for a range of different body builds and bust sizes. This will increase patient uptake and use of the device, leading to improved therapeutic outcomes for a greater number of patients.

In tests, it has been shown that the support vest 100 described herein provides an even and consistent hold force. The support vest has been tested on a standard size control system, manufactured by CETME and found to provide a consistent hold force over a range of different stretch positions.

In a recent clinical evaluation of 43 patients, the patients were given the support vest described herein and asked to rate its comfort on a scale of 1-5 (low to high comfort). The median score for the 43 patients was 4.

In a recent real world clinical deployment involving a small number of patients, the support vest described herein was found to be safe and effective.

In an exemplary implementation of the support vest 100, the central elongate portion 102 is made of a mix of polyamide and elastane in the ranges of 50-70% polyamide and 30-50% elastane. In one specific implementation of the support vest 100, the central elongate portion 102 is made of a mix of polyamide and elastane in the ratio 59% polyamide and 41% elastane. Where the first peripheral portion 104 and the second peripheral portion 106 are also made of a resilient material, they may also be made of a polyamide and elastane mix having the above ratios. In one example, the central elongate portion 102 has a weight of 500 g/m². The central elongate portion 102 may have a double wall construction resulting in an internal space. This internal space may be filled with a polyester wadding.

The materials used for the body contacting parts of the support vest 100 ensure that the vest 100 is breathable and acts to wick away moisture, making it more comfortable to wear than existing support systems. An additional advantage of using the materials described above is that the whole support vest 100 can be washed (at high temperature) without damage or significant degradation of the structure.

The support vest 100 also comprises an adjustable support strap 114 extending from an upper edge of the central elongate portion 102. The adjustable support strap 114 is configured to set and maintain a vertical position of the vest around the patent's thorax. The adjustable support strap 114 comprises a back yoke with two shoulder extensions arranged to pass over the patient's should and connect to a pair of connecting strips 116. The connecting strips 116 extend from the upper surface of the central elongate portion, first peripheral portion or second peripheral portion. In the design of Figure 1a, one of the connecting strips 116 extends from the upper surface of the central elongate portion and the other connecting strips 116 extends from the upper surface of the second peripheral portion 106. As can be seen in the Figures, the adjustable support strap has a tapered shape. This shape, combined with the depth and position of the central elongate portion 102 ensures that there is no material located over boney prominences, such as the shoulder blades. This means that no force is exerted on these areas, reducing the risk of a pressure injury. A further advantage of the design of the support vest 100 is that it can be applied or removed by a healthcare worker, while the patient is supine (lying face up). The low profile of the support vest 100 allows it to be easily slid underneath a supine patient. For example, to remove the support vest 100, a healthcare worker only needs to undo the support strap 114 from the connecting strips 116 and release the attachment surfaces, both of which are easily accessible from the front of the support vest 100.

The connection between the shoulder extensions of the adjustable support strap 114 and the connecting strips 116 is a releasable connection designed to be easily fastened and unfastened by the patient without assistance. For example, the connection may use a contact or touch based fastener, such as a hook and loop fastener (e.g. Velcro^{®}) as previously described. The adjustable support strap 114 may also made of a resilient material, for example the polyamide and elastane mix described above. The adjustable support strap 114 allows the position of the support vest 100 on the user's thorax to be set and maintained correctly and accurately and to be quickly adjusted by the patient if necessary.

The support vest 100 may optionally comprise one or more removable inserts (not shown) arranged substantially perpendicular to the elongate direction of the central elongate portion 102 when inserted and configured to lift a portion of the support vest 100 away from the body of the patient. Pockets may be provided in the support vest 100 to accommodate these removable inserts. Lifting the support vest 100 away from the body of the patient Improves air circulation over the wound site. It also lifts the support vest 100 and any other garments so as to leave drains undisturbed and un-restricted.

Figure 1b shows the same support vest 100 as in Figure 1a with labelled dimension arrows A-H. The corresponding dimensions are indicated below in table 1. These are provided merely as examples of the various sizes which may be employed in the support vest 100 and are not intended to be limiting on the scope of protection, which is defined in the appended claims.

**Table 1.**

| | | | XS | S | M | L | XL | XXL | Tol +/-cm |
|---|---|---|---|---|---|---|---|---|---|
| | CHEST SIZING IN CENTIMETRES | | 80 - 92 | 92 - 102 | 102 - 112 | 112 - 122 | 122 - 132 | 132 - 142 | |
| | CHEST SIZING IN INCHES | | 32 - 36 | 36 - 40 | 40 - 44 | 44 - 48 | 48 - 52 | 52 - 56 | |
| A | TOTAL LENGTH TIP TO TIP | | 102 | 110 | 118 | 126 | 134 | 142 | 2 |
| | MINIMUM STRETCH LENGTH TIP TO TIP | | 114 | 124 | 134 | 142 | 150 | 158 | |
| B | ACTIVE STRAP LENGTH | | 66 | 74 | 82 | 90 | 98 | 106 | |
| C | DEPTH OF STRAP 20 CMS | | 20 | 20 | 20 | 20 | 20 | 20 | 1 |
| D | GLOVE LENGTH | | 16 | 16 | 16 | 16 | 16 | 16 | 1 |
| E | ADJUSTABLE VELCRO LOOP PAD LENGTH | | 20 | 20 | 20 | 20 | 20 | 20 | 1 |
| F | LOWER SHOULDER STRAP LENGTH | | 14 | 14 | 14 | 14 | 14 | 14 | 1 |
| G | BACK YOKE LENGTH | | 34 | 36 | 38 | 40 | 42 | 44 | 1 |
| H | BACK YOKE WIDTH | | 27 | 28 | 29 | 30 | 31 | 32 | 1 |

As can be appreciated from the difference between the "total length tip to tip" and "minimum stretching length tip to tip" the length of the support vest can increase by at least 11%, providing a wide range of size adjustment and a wide range of support force adjustment without the need to re-set or adjust straps or buckles.

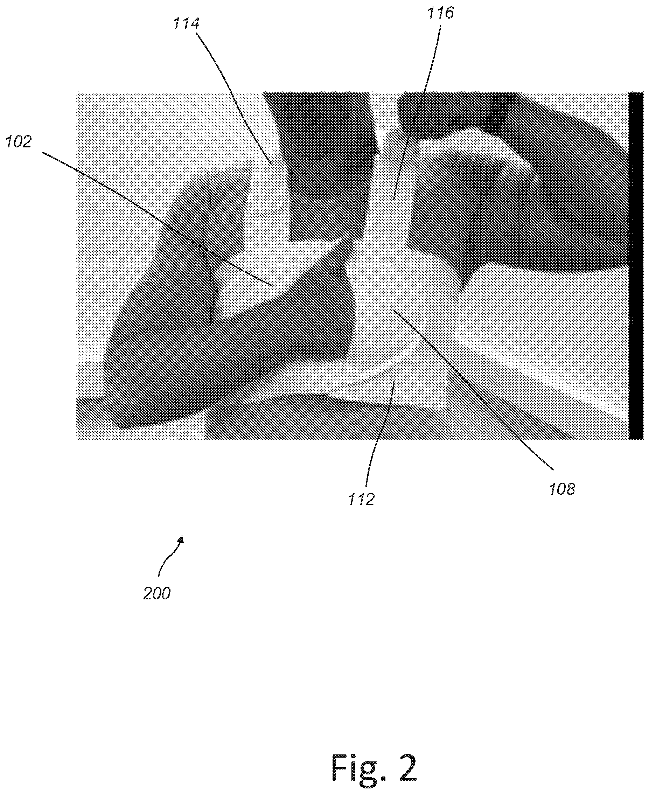

Figure 2 shows an image 200 of the support vest 100 of Figure 1a being demonstrated. As can be seen, the pocket 108 on the first peripheral portion 104 is positioned so as to allow convenient and independent use by the patient. The adjustable nature of the support straps is also shown. As can also be seen, the position of the pocket 108 is off centre with respect to the thorax of the patient when the vest is worn by the patient. This is advantageous since the wound site is typically over the sternum, i.e. centrally located. In prior art support systems in which the buckles or adjusting straps are also centrally located there is an increased risk of infection from hands touching the wound site when the support system is attached or adjusted. Touching or applying increased pressure to the wound site is also painful for the patient, which may lead to a reluctance on the part of patients to wear and/or adjust the support system, leading to poorer therapeutic outcomes. There is also a risk of lateral strain occurring with front fastening buckles/clasps if these become stuck. These problems are alleviated by the support vest 100 of the present invention, since the fastening arrangement is located off centre and therefore away from the likely wound site. The first peripheral portion 104 is also arranged so as to overlap and contact the second peripheral portion 106, further reducing the risk of contacting the wound site. The patient's hand is also enclosed inside the pocket 108, reducing the risk of infection through contact with hands.

### References

1. Vos RJ, Van Putte BP, Kloppenburg GTL. Prevention of deep sternal wound infection in cardiac surgery: a literature review. J Hosp Infect. 2018 Dec;100(4):411-420. doi: 10.1016/j.jhin.2018.05.026. Epub 2018 Jun 7. PMID: 29885873.
2. Gorlitzer M, Wagner F, Pfeiffer S, Folkmann S, Meinhart J, Fischlein T, Reichenspurner H, Grabenwoeger M. Prevention of sternal wound complications after sternotomy: results of a large prospective randomized multicentre trial. Interact Cardiovasc Thorac Surg. 2013 Sep;17(3):515-22. doi: 10.1093/icvts/ivt240. Epub 2013 Jun 11. PMID: 23760221; PMCID: PMC3745148.
3. Celik, S. et al. (2011) "Sternal Celic dehiscence in patients with moderate and severe chronic obstructive pulmonary disease undergoing cardiac surgery: The value of supportive thorax vests", The journal of Thoracic and Cardiovascular Surgery, 141(6), pp.1398-1402.doi10.1016/j.jtcys.2011.01.042

### Reference numerals

**100** support vest
**102** central elongate portion
**104** first peripheral portion
**106** second peripheral portion
**108** pocket
**110** attachment surface
**112** co-operating attachment surface
**114** adjustable support strap
**116** connecting strips
**200** image

## Claims

1. A post-surgical thoracic support vest (100) comprising:
a resilient central elongate portion (102) configured to be wrapped around the thorax of a patient;
a first peripheral portion (104) disposed at a first end of the central elongate portion, the first peripheral portion comprising:
an attachment surface (110) disposed on an inner surface of the first peripheral portion;
a second peripheral portion (106) disposed at a second end of the central elongate portion, wherein the second peripheral portion comprises a co-operating attachment surface (112) on an outer surface of the second peripheral portion and is configured to be releasably secured to the first peripheral portion via the attachment surfaces, **characterised in that** the first peripheral portion further comprises a pocket (108) disposed on an outer surface of the first peripheral portion, the pocket arranged so as to allow the patient to insert at least a part of a hand into the pocket, wherein the position of the pocket is off centre with respect to the thorax of the patient when the vest is worn by the patient.

2. The post-surgical thoracic support vest of claim 1, further comprising an adjustable support strap extending from an upper edge of the central elongate portion and configured to set and maintain a vertical position of the vest around the patent's thorax.

3. The post-surgical thoracic support vest (100) of claim 1, wherein the adjustable support strap is arranged to pass over the shoulders of the patient and be releasably secured to a pair of connecting strips (116) which extend from the upper surface of the central elongate portion, first peripheral portion or second peripheral portion.

4. The post-surgical thoracic support vest (100) of any preceding claim, wherein the attachment surface and the co-operating attachment surface together comprise a touch based fastening arrangement.

5. The post-surgical thoracic support vest (100) of claim 3, wherein the touch based fastening arrangement is a hook and loop fastener.

6. The post-surgical thoracic support vest (100) of any preceding claim, wherein an opening of the pocket is arranged perpendicular to the elongate direction of the central elongate portion.

7. The post-surgical thoracic support vest (100) of any preceding claim, wherein the pocket is configured to allow the patient to attach and release the attachment surfaces using a single hand while wearing the vest.

8. The post-surgical thoracic support vest (100) of any preceding claim, wherein the central elongate portion is made of a mix of polyamide and elastane in the ranges of 50-70% polyamide and 30-50% elastane.

9. The post-surgical thoracic support vest (100) of claim 8, wherein the central elongate portion is made of a mix of polyamide and elastane in the ratio 59% polyamide and 41% elastane.

10. The post-surgical thoracic support vest (100) of any preceding claim, wherein the first peripheral portion and second peripheral portion are made of a resilient material.

11. The post-surgical thoracic support vest (100) of any preceding claim, wherein the vest further comprises one or more removable inserts arranged substantially perpendicular to the elongate direction of the central elongate portion when inserted and configured to lift a portion of the vest away from the body of the patient.

## Patentansprüche

1. Weste (100) zur postchirurgischen Thoraxunterstützung, umfassend:
einen elastischen zentralen länglichen Abschnitt (102), der dazu konfiguriert ist, um den Thorax eines Patienten gewickelt zu sein;
einen ersten Umfangsabschnitt (104), der an einem ersten Ende des zentralen länglichen Abschnitts angeordnet ist, wobei der erste Umfangsabschnitt Folgendes umfasst:
eine Anbringungsfläche (110), die auf einer Innenfläche des ersten Umfangsabschnitts angeordnet ist;
einen zweiten Umfangsabschnitt (106), der an einem zweiten Ende des zentralen länglichen Abschnitts angeordnet ist, wobei der zweite Umfangsabschnitt eine zusammenwirkende Anbringungsfläche (112) an einer Außenfläche des zweiten Umfangsabschnitts umfasst und dazu konfiguriert ist, über die Anbringungsflächen lösbar an dem ersten Umfangsabschnitt gesichert zu werden, **dadurch gekennzeichnet, dass** der erste Umfangsabschnitt ferner eine Tasche (108) umfasst, die an einer Außenfläche des ersten Umfangsabschnitts angeordnet ist, wobei die Tasche so angeordnet ist, dass sie es dem Patienten ermöglicht, mindestens einen Teil einer Hand in die Tasche einzuführen, wobei die Position der Tasche in Bezug auf den Thorax des Patienten außermittig ist, wenn die Weste von dem Patienten getragen wird.

2. Weste zur postchirurgischen Thoraxunterstützung nach Anspruch 1, ferner umfassend einen einstellbaren Stützriemen, der sich von einem oberen Rand des zentralen länglichen Abschnitts erstreckt und dazu konfiguriert ist, eine vertikale Position der Weste um den Thorax des Patienten einzustellen und aufrechtzuerhalten.

3. Weste (100) zur postchirurgischen Thoraxunterstützung nach Anspruch 1, wobei der einstellbare Stützriemen dazu angeordnet ist, über die Schultern des Patienten zu verlaufen und lösbar an einem Paar Verbindungsstreifen (116) gesichert zu sein, die sich von der oberen Fläche des zentralen länglichen Abschnitts, des ersten Umfangsabschnitts oder des zweiten Umfangsabschnitts erstrecken.

4. Weste (100) zur postchirurgischen Thoraxunterstützung nach einem vorhergehenden Anspruch, wobei die Anbringungsfläche und die zusammenwirkende Anbringungsfläche zusammen eine berührungsbasierte Befestigungsanordnung umfassen.

5. Weste (100) zur postchirurgischen Thoraxunterstützung nach Anspruch 3, wobei die berührungsbasierte Befestigungsanordnung ein Klettverschluss ist.

6. Weste (100) zur postchirurgischen Thoraxunterstützung nach einem vorhergehenden Anspruch, wobei eine Öffnung der Tasche senkrecht zur länglichen Richtung des zentralen länglichen Abschnitts angeordnet ist.

7. Weste (100) zur postchirurgischen Thoraxunterstützung nach einem vorhergehenden Anspruch, wobei die Tasche dazu konfiguriert ist, es dem Patienten zu ermöglichen, die Anbringungsflächen unter Verwendung einer einzigen Hand anzubringen und freizugeben, während er die Weste trägt.

8. Weste (100) zur postchirurgischen Thoraxunterstützung nach einem vorhergehenden Anspruch, wobei der zentrale längliche Abschnitt aus einer Mischung aus Polyamid und Elastan in den Bereichen von 50-70 % Polyamid und 30-50 % Elastan hergestellt ist.

9. Weste (100) zur postchirurgischen Thoraxunterstützung nach Anspruch 8, wobei der zentrale längliche Abschnitt aus einer Mischung aus Polyamid und Elastan im Verhältnis von 59 % Polyamid und 41 % Elastan hergestellt ist.

10. Weste (100) zur postchirurgischen Thoraxunterstützung nach einem vorhergehenden Anspruch, wobei der erste Umfangsabschnitt und der zweite Umfangsabschnitt aus einem elastischen Material hergestellt sind.

11. Weste (100) zur postchirurgischen Thoraxunterstützung nach einem vorhergehenden Anspruch, wobei die Weste ferner einen oder mehrere entfernbare Einsätze umfasst, die im Wesentlichen senkrecht zur länglichen Richtung des zentralen länglichen Abschnitts angeordnet sind, wenn sie eingeführt sind, und dazu konfiguriert sind, einen Abschnitt der Weste vom Körper des Patienten wegzuheben.

## Revendications

1. Gilet de soutien thoracique post-chirurgical (100) comprenant :
une partie centrale allongée élastique (102) conçue pour être enroulée autour du thorax d'un patient ;
une première partie périphérique (104) disposée au niveau d'une première extrémité de la partie centrale allongée, la première partie périphérique comprenant :
une surface de fixation (110) disposée sur une surface interne de la première partie périphérique ;
une seconde partie périphérique (106) disposée au niveau d'une seconde extrémité de la partie centrale allongée, ladite seconde partie périphérique comprenant une surface de fixation coopérante (112) sur une surface externe de la seconde partie périphérique et étant conçue pour être fixée de manière amovible à la première partie périphérique par l'intermédiaire des surfaces de fixation, **caractérisé en ce que** la première partie périphérique comprend en outre une poche (108) disposée sur une surface externe de la première partie périphérique, la poche étant agencée de manière à permettre au patient d'insérer au moins une partie d'une main dans la poche, ladite position de la poche étant décentrée par rapport au thorax du patient lorsque le gilet est porté par le patient.

2. Gilet de soutien thoracique post-chirurgical selon la revendication 1, comprenant en outre une sangle de soutien réglable s'étendant à partir d'un bord supérieur de la partie centrale allongée et conçue pour définir et maintenir la position verticale du gilet autour du thorax du patient.

3. Gilet de soutien thoracique post-chirurgical (100) selon la revendication 1, ladite sangle de soutien réglable étant agencée pour passer sur les épaules du patient et être fixée de manière amovible à une paire de bandes de raccordement (116) qui s'étendent à partir de la surface supérieure de la partie centrale allongée, de la première partie périphérique ou de la seconde partie périphérique.

4. Gilet de soutien thoracique post-chirurgical (100) selon l'une quelconque des revendications précédentes, ladite surface de fixation et ladite surface de fixation coopérante comprenant ensemble un agencement de fixation tactile.

5. Gilet de soutien thoracique post-chirurgical (100) selon la revendication 3, ledit agencement de fixation tactile étant une bande de fixation velcro.

6. Gilet de soutien thoracique post-chirurgical (100) selon l'une quelconque des revendications précédentes, une ouverture de la poche étant agencée perpendiculairement à la direction allongée de la partie centrale allongée.

7. Gilet de soutien thoracique post-chirurgical (100) selon l'une quelconque des revendications précédentes, ladite poche étant conçue pour permettre au patient de fixer et de libérer les surfaces de fixation à l'aide d'une seule main tout en portant ledit gilet.

8. Gilet de soutien thoracique post-chirurgical (100) selon l'une quelconque des revendications précédentes, ladite partie centrale allongée étant constituée d'un mélange de polyamide et d'élasthanne compris dans les plages de 50 à 70 % de polyamide et de 30 à *50* % d'élasthanne.

9. Gilet de soutien thoracique post-chirurgical (100) selon la revendication 8, ladite partie centrale allongée étant constituée d'un mélange de polyamide et d'élasthanne selon un rapport de 59 % de polyamide et 41 % d'élasthanne.

10. Gilet de soutien thoracique post-chirurgical (100) selon l'une quelconque des revendications précédentes, ladite première partie périphérique et ladite seconde partie périphérique étant constituées d'un matériau élastique.

11. Gilet de soutien thoracique post-chirurgical (100) selon l'une quelconque des revendications précédentes, ledit gilet comprenant en outre un ou plusieurs inserts amovibles agencés sensiblement perpendiculairement à la direction allongée de la partie centrale allongée lorsqu'ils sont insérés et conçus pour soulever une partie du gilet loin du corps du patient.
